# EUROPÄISCHE PATENTANMELDUNG

(11) **EP 1 136 120 A1**
(43) Veröffentlichungstag der Anmeldung: **26.09.2001**
(21) Anmeldenummer: 01250102.9
(22) Anmeldetag: 22.03.2001
(51) Int. Cl.: B01J 27/198, C07C 51/215, C07C 57/145, C07C 253/24

(54) **Mischkatalysator aus Oxovanadiumdiphosphat und weiteren Komponenten**

(30) Priorität: 23.03.2000 DE 10015365
(71) Anmelder: Institut für Angewandte Chemie Berlin-Adlershof E.V., 12489 Berlin (DE)
(72) Erfinder: Wolf, Gert-Ulrich, 15827 Blankenfelde (DE); Kubias, Bernd, 12489 Berlin (DE); Martin, Andreas, 12524 Berlin (DE); Schülke, Ulrich, 12623 Berlin (DE)
(74) Vertreter: Walter, Wolf-Jürgen

(57) **Zusammenfassung**

Die Erfindung betrifft einen Mischkatalysator aus (VO)₂P₂O₇ und weiteren Komponenten, der über einen VOHPO₄ • 0,5H₂O-Katalysator-precursor hergestellt werden kann. Die Herstellung erfolgt durch Reaktion von VO(H₂PO₄)₂ mit einem Metalloxid, Metallhydroxid, Metalloxidhydroxid oder einem Gemisch davon oder eines Gemisches von Me(H₂PO₄)₂ und VO₂ unter hydrothermalen Bedingungen in einem geschlossenen System in Anwesenheit von 0,5 bis 40 Mol-% Wasser bei einem Druck von 1,05 bis 6 bar und bei einer Temperatur im Bereich von 100 bis 150°C über einen Zeitraum von 0,5 bis 100 Stunden zu VOHPO₄ • 0,5H₂O und einem Metallphosphatprodukt. Das erhaltene Gemisch wird bei Temperaturen von 380 bis 700°C calciniert wird bis zur Bildung des Katalysators.

## Beschreibung

Die Erfindung betrifft einen Mischkatalysator aus (VO)₂P₂O₇ und weiteren Komponenten, der über einen VOHPO₄ · 0,5H₂O-Katalysatorprecursor hergestellt werden kann.

Oxovanadiumdiphosphat-Katalysatoren werden zur katalytischen Herstellung von Maleinsäureanhydrid durch Oxidation von C₄-Kohlenwasserstoffen, insbesondere von n-Butan, technisch verwendet. Die katalytischen Eigenschaften dieses Katalysators werden vor allem durch folgende Faktoren bestimmt:
Verfahren zur Herstellung des Precursors,
Verfahren zur Einstellung des P:V-Verhältnisses und
Verfahren zur Aktivierung und Konditionierung des Precursors.

Für die Herstellung des Precursors (VOHPO₄ · 0,5H₂O) sind verschiedene Verfahren bekannt, so z.B. die Reduktion von V₂O₅ mit organischen Lösungsmitteln, Umsetzung mit Phosphor-säure und nachfolgender Calcinierung (JP-Sho 57-8761/1982 und JP-Hei 1-50455/1989). Weiterhin ist die Herstellung beschrieben durch Reduktion von V₂O₅ mit HCl, Oxalsäure; Hydrazin u.a. in Gegenwart von phosphoriger Säure und anschließende Calcinierung (US-A-4085122).

Grundsätzlich lassen sich die bekannten Herstellungsverfahren auf 3 Wege zurückführen:
1) Reduktion einer V⁵⁺-Verbindung (z.B. V₂O₅) in Wasser, Zugabe von Phosphorsäure und Isolierung des Precursors (Chem. Rev., **1988,** 88, 55).
2) Reduktion einer V⁵⁺-Verbindung in einem organischen Lösungsmittel, Zugabe von phosphoriger Säure und Abtrennung des Precursors (Appl. Catal., **1988,** 38, 193).
3) Herstellung von VOPO₄ und Reduzierung zu VOHPO₄ · 0,5H₂O entweder in Wasser oder in einem organischen Lösungsmittel (I. Matsuura in "New Developments in Selective Oxidation by Heterogeneous Catalysis", ed. P. Ruiz and B. Delmon, Elsevier Science Publ., Amsterdam, **1992,** p. 247).

Diese grundsätzlichen Verfahren werden zur Herstellung von VOHPO₄ · 0,5H₂O häufig variiert, um zu einem optimalen Precursor für einen selektiven und aktiven Katalysator zu gelangen.

Aus der EP-A-804963 sind Mischkatalysatoren aus Oxovanadiumdiphosphat bekannt, die im Fließbett durch Erhitzung des Precursors auf 380-600 °C bei Überdruck, Halten der erreichten Temperatur und Abkühlen des aktivierten Katalysators hergestellt werden.

Weiterhin ist aus WO89/55226 ein Herstellungsverfahren eines VPO-Mischkatalysators mit anderen Kationen bekannt, bei dem das VOHPO₄-Halbhydrat aus einer auch andere Kationen enthaltenden Lösung ausgefällt und calciniert wird.

Die DE 198 25 389 A1 beschreibt eine hydrothermale Herstellung eines Oxovanadiumdiphosphat-Katalysators, der mit verschiedenen Kationen dotiert sein kann, jedoch keine weitere Phosphatphase enthält.

Aufgabe der Erfindung ist die Bereitstellung eines aktiven Vanadiumpyrophosphat-Katalysators über einen neuen und einfachen Herstellungsweg.

Es wurde nun überraschenderweise gefunden, dass durch hydrothermale Behandlung eines Gemisches von VO(H₂PO₄)₂ und einem Metalloxid, einem Metallhydroxid und einem Metalloxidhydroxid oder einem Gemisch davon oder eines Gemisches von Metall-bis-dihydrogenphosphat und VO₂ in einem geschlossenen System in Anwesenheit von 0,5 bis 40 Mol-% Wasser bei einem Druck von 1,05 bis 6 bar und bei einer Temperatur im Bereich von 100 bis 150°C über einen Zeitraum von 0,5 bis 100 Stunden in VOHPO₄ · 0,5H₂O und ein Metallphosphatprodukt überführt werden kann, wobei das Molverhältnis von Metalloxid zu VO(H₂PO₄)₂ im Bereich von 0,9:1 bis 1,1:2 liegt, und das erhaltene Gemisch bei Temperaturen von 380 bis 700°C zu wasserfreien kristallinen Mischkatalysatoren der Formel (I)

(VO)₂P₂O₇ · A (I)

umgewandelt werden kann, worin A die Bedeutung Meₓ(PO₄)_{y} oder Me₂P₂O₇ hat und x= 1 bis 3 und y= 1, 2 und 4 darstellen, und wobei jeweils Me ein Metall ist, ausgewählt aus der Gruppe, die aus Fe, La, Lanthanide, Sb, Al, Bi, Zr, V, Zn, Mn, Cu, Sn, Ti besteht.

Das Metallphosphatprodukt kann in Abhängigkeit vom eingesetzten Metall kristallwasserhaltig sein.

Bevorzugte Metalle sind Lanthan, Titanium, Zink, Kupfer, Aluminium, Zirkonium, Bismut und Eisen in Form ihrer Oxide oder Hydroxide.

Nach einer bevorzugten Ausführungsform der Erfindung werden bei der Herstellung die VO(H₂PO₄)₂-Ausgangsmaterialien mit dem Metalloxid, -hydroxid oder -oxidhydroxid bzw. einem Gemisch von Verbindungen mehrerer Metalle intensiv gemischt. Die Gemische werden vorzugsweise zu Tabletten verpresst und direkt oder als Splitt der nachfolgenden hydrothermalen Behandlung unterworfen.

Bevorzugte Metalle sind Lanthan, Lanthanide, Titanium, Zink, Kupfer, Aluminium, Zirkonium, Bismut, Vanadium und Eisen in Form ihrer Oxide oder Hydroxide.

Bei der thermischen Behandlung sind Temperaturen von 450 bis 550 °C im Inertgasstrom bevorzugt.

Durch das verwendete Metalloxid, -hydroxid oder -oxidhydroxid wird die Kristallitgröße und -morphologie des gebildeten Halbhydrates VOHPO₄ · 0,5H₂O und die Größe der Oberfläche des Precursorgemisches VOHPO₄ · 0,5H₂O/Me-phosphat und des Calcinierungsproduktes der Formel (I) wesentlich mitbestimmt.

Die Kristallitgröße läßt sich aus der Halbwertsbreite der Röntgenreflexe berechnen (gemäß Acta Metall. 1, 22(1922). Die spezifische Oberfläche wird mit Hilfe der BET-Methode bestimmt (Z.Anal. Chem. 238. 187(1968).

Auf diese Weise lassen sich Katalysatoroberflächen von 0,2 m²/g bei Einsatz von ZnO beispielsweise und bis zu 40 m²/g bei Einsatz von La₂O₃ beispielsweise gezielt einstellen.

Die erfindungsgemäß hergestellten Katalysatoren können bei der Oxidation von Alkanen, wie z.B der Selektivoxidation von Butan zu Maleinsäure sowie bei der Ammoxidation von Toluol zu Benzonitril oder von Toluol zu Benzaldehyd eingesetzt werden.

Die Erfindung soll nachstehend durch Beispiele näher erläutert werden.

### Herstellung des VO(H₂PO₄)₂-Ausgangsproduktes

9,2 g Phosphorige Säure und 34,6 g einer 85%igen Phosphorsäure werden mit 100 ml Wasser verdünnt und auf 90°C erhitzt. In die heiße Lösung werden unter intensivem Rühren innerhalb von 1,5 Stunden portionsweise 18,19 g Vanadiumpentoxid eingetragen und anschließend die Lösung 4 Stunden bei 95°C gerührt. Das Gesamtvolumen der Lösung wurde während dieser Zeit konstant bei 150 ml gehalten. Nach Filtrieren der abgekühlten tiefblauen Lösung wird das Filtrat in einer mit Teflonfolie ausgelegten Schale langsam bis zur Trockene eingedampft. Das so gewonnene Rohprodukt wird in 50 ml Wasser suspendiert und durch langsames Eindampfen vollständig durchkristallisiert. Nach Trocknen der Kristalle im Wärmeschrank bei 130°C betrug die Ausbeute 49,9 g (= 95,6% d. Th.).

Das Röntgenpulverdiagramm der Kristalle besteht nur aus den Reflexen des VO(H₂PO₄)₂.

Für die Herstellung der Precursoren wurde das VO(H₂PO₄)₂-Ausgangsmaterial in einer Achatkugelmühle aufgemahlen.

### Beispiel 1

10,44 g VO(H₂PO₄)₂ wurden zusammen mit 5,87 g La₂O₃ durch Mörsern intensiv vermischt und das Gemisch unter einem Druck von 9 MPa zu Tabletten gepresst. Die Tabletten wurden anschließend zu Splitt gebrochen und die Kornfraktion D=1,25-2,5 mm ausgesiebt.

11,87 g Splitt der Kornfraktion 1,25 - 2,5 mm und ein mit 1,50 g Wasser gefülltes Reagenzglas wurden in einer Glasampulle eingeschlossen und bei 130°C erhitzt. Nach einer Reaktionszeit von 30 Minuten waren die Splittkörner zu einem blauen Pulver zerfallen. Nach 66 Stunden wurde die Ampulle geöffnet und das überschüssige Wasser durch 2-stündiges Erhitzen bei 130°C entfernt. Die Ausbeute betrug 11,32 g.

Der so hergestellte Precursor bestand aus einem VOHPO₄ · 0,5H₂O / LaPO₄-Gemisch von sehr geringer Kristallinität. Die spezifische Oberfläche (BET-Oberfläche) betrug 37,1 m²/g.

Der Katalysator, (VO)₂P₂O₇ · LaPO₄ wurde durch 4-stündiges Erhitzen des Precusors bei 500°C im N₂-Strom hergestellt. BET-Oberfläche= 38,4 m²/g

### Beispiel 2

10,44 g VO (H₂PO₄)₂-Ausgangsprodukt wurden mit 9,32 g Bi₂O₃ durch Mörsern intensiv gemischt und die Mischung wie in Beispiel 1 zu Splitt weiterverarbeitet.

15,00 g Splitt (D=1,25-2,5 mm) und ein Reagenzglas mit 1,50 g Wasser wurden in einer Ampulle eingeschmolzen 96 Stunden bei 140°C erhitzt. Nach öffnen der Ampulle und Trocknen des Erhitzungsprodukts bei 130°C wurden 14,0 g Precursor erhalten, der aus einem Gemisch von VOHPO₄ · 0,5H₂O und BiPO₄ bestand. BET-Oberfläche= 2,9 m²/ g.

5,00 g des Gemisches wurden im N₂-Strom bei 500°C erhitzt. Dabei wurden 4,67 g des Katalysators VO)₂P₂O₇ · BiPO₄ erhalten. BET-Oberfläche= 3,3 m²/ g.

### Beispiel 3

10,44 g VO(H₂PO₄)₂-Ausgangsprodukt und 2,88 g reaktives TiO₂ wurden gemeinsam gemörsert, und wie in Beispiel 1 zu Splitt verarbeitet. 9,49 g des Splitts (D=1,25-2,5 mm) und ein mit 1,40 g Wasser gefülltes Reagenzglas wurden in einer Glasampulle ein-geschlossen und bei 140°C 67 Stunden erhitzt. Die abgekühlte Ampulle wurde nach öffnen zur Entfernung von nicht umgesetztem Wasser 2,5 Stunden bei 130°C erhitzt.

Der grüngefärbte Rückstand von 9,33 g bestand aus einem Gemisch von VOHPO₄ · 0,5H₂O und Titanphosphat. BET-Oberfläche=13,9 m²/g.

9,00 g dieses Gemisches wurden 4 Stunden im Stickstoffstrom bei 500°C erhitzt. Dabei entstanden 8,73 g des Katalysators VO)₂P₂O₇ · Ti-Phosphat. BET-Oberfläche= 26,7 m²/ g.

### Beispiel 4

5,22 g VO(H₂PO₄)₂-Ausgangsprodukt und 1,83 g ZnO wurden wie in Beispiel 1 zu Splitt verarbeitet 3,00 g Splitt (D=1,25-2,5 mm) wurden zusammen mit einem mit 0,35 g Wasser gefüllten Reagenzglas in einer geschlossenen Ampulle 24 Stunden bei 130°C erhitzt. Nach Öffnen der Ampulle wurde das blaugefärbte Erhitzungsprodukt eine Stunde bei 130°C getrocknet. Die Ausbeute betrug 2,99 g. XRD-Untersuchungen ergaben, dass der Precursor aus einem Gemisch von VOHPO₄ · 0,5H₂O und ZnHPO₄ bestand; BET-Oberfläche= 0,49 m²/ g.

2,50 g des Precursors wurden 2 Stunden bei 500°C in Stickstoff erhitzt. Auf diese Weise wurden 2,29 g des Katalysators VO)₂P₂O₇ · Zn₂P₂O₇ erhalten.
BET-Oberfläche = 0,56 m²/ g.

### Beispiel 5

5,22 g VO(H₂PO₄)₂-Ausgangsprodukt und 2,45 g ZnO wurden nach intensivem Vermischen und Mörsern wie in Beispiel 1 zu Splitt verarbeitet. 5,81 g Splitt wurden mit 0,64 g Wasser durchfeuchtet und 48 Stunden bei 130°C erhitzt.

Die Ausbeute nach Trocknen betrug 5,94 g Precursor, der aus einem Gemisch von VOHPO₄ · 0,5H₂O und Zn₃(PO₄)₂ · 4H₂O bestand.
BET-Oberfläche= 1,27 m²/ g.

Durch 2-stündiges Erhitzen von 3,51 g Precursor bei 500°C im Inertgasstrom wurden 3,11 g Katalysator VO)₂P₂O₇ · Zn₃(PO₄)₂ erhalten. BET-Oberfläche= 3,7 m²/ g.

### Beispiel 6

5,94 g Zn(H₂PO₄)₂ · 2H₂O wurden mit 1,66 g VO₂ intensiv vermischt und gemörsert und wie in Beispiel 1 zu Splitt verarbeitet. 3,00 g Splitt wurden in einer geschlossenen Ampulle 48 Stunden bei 130°C erhitzt. Dabei bildete sich ein grüngefärbtes Produkt. Bei 1-stündigem Trocknen bei 130°C trat ein Gewichtsverlust von 0,32 g auf. Röntgenographisch wurde in diesem Precursor ausschließlich VOHPO₄ · 0,5H₂O nachgewiesen; die zinkhaltige Komponente lag als amorphe Phase vor.
BET-Oberfläche= 0.40 m²/ g.

### Beispiel 7

5,22 g VO(H₂PO₄)₂-Ausgangsprodukt wurden mit 1,51 g CuO innig gemischt, gemörsert und zu Splitt verarbeitet. 5,04 g des Splitts wurden mit 0,50 g H₂O durch 40-stündiges Erhitzen bei 130°C in einer geschlossenen Ampulle umgesetzt. Nach Trocknen bei 130°C hinterblieben 5,01 g tiefblauer Precursor, der aus VOHPO₄ · 0,5H₂O und CuHPO₄ · H₂O bestand. BET-Oberfläche= 0,42 m²/ g.

Beim 2-stündigen Erhitzen von 3,23 g Precursor bei 500°C wurden 2,83 g des Katalysators (VO)₂P₂O₇ · Cu₂P₂O₇ erhalten.
BET-Oberfläche= 9,3 m²/g.

### Beispiel 8

10,44 g VO(H₂PO₄)₂-Ausgangsprodukt und 5,83 g Sb₂O₃ wurden nach ausgiebigem Mischen und Mörsern wie in Beispiel 1 zu Splitt verarbeitet. 15,00 g Splitt wurden zusammen mit einem mit 1,50 g Wasser gefüllten Gefäß in einer geschlossenen Glasampulle 48 Stunden bei 140°C erhitzt. Nach Trocknen des Erhitzungsproduktes bei 130°C wurden 14,50 g Precursor, bestehend aus VOHPO₄ · 0,5H₂O und SbPO₄, erhalten. BET-Oberfläche= 1,61 m²/g.

5,00 g Precursor führten bei Calcinieren bei 500°C im Inertgas zu 4,86 g Katalysator (VO)₂P₂O₇ · SbPO₄.
BET-Oberfläche= 3,0 m²/g.

### Beispiel 9

5,22 g VO(H₂PO₄)₂-Ausgangsprodukt und 1,44 g Fe₂O₃ wurden wie in Beispiel 1 durch intensives Mörsern vermischt und zu Splitt verarbeitet. 3,00 g Splitt wurden gemeinsam mit einem mit 0,40 g H₂O gefüllten Reagenzglas in einer geschlossenen Ampulle bei 130°C 73 Stunden erhitzt. Nach öffnen der Ampulle wurde überschüssiges Wasser bei 130°C entfernt und 3,05 g Precursor erhalten, der aus VOHPO₄ · 0,5H₂O und FePO₄ · 2H₂O bestand.
BET-Oberfläche= 3,4 m²/ g.

Durch 2-stündiges Erhitzen des Precursors bei 500°C im Stickstoffstrom wurde der Katalysator (VO)₂P₂O₇ · FePO₄ erhalten. BET-Oberfläche= 7,3 m²/ g.

### Beispiel 10

5,22 g VO(H₂PO₄)₂-Ausgangsprodukt wurden mit 2,04 g γ- Al₂O₃ gemischt, intensiv gemörsert und wie in Beispiel 1 zu Splitt verarbeitet. 5,00 g Splitt wurden zusammen mit in einem gesonderten Gefäß befindlichen 0,60 g Wasser in einer geschlossenen Ampulle bei 130°C erhitzt. Bereits nach kurzer Zeit trat eine Verfärbung auf. Nach 48-stündiger Reaktionszeit und Trocknen waren 4,82 g Precursor entstanden, der aus VOHPO₄ · 0,5H₂O und AlPO₄ bestand. BET-Oberfläche= 0,54 m²/ g.

2-stündiges Erhitzen in einem Stickstoffstrom bei 500°C führte zum Katalysator (VO)₂P₂O₇ · AlPO₄.
BET-Oberfläche= 1,57 m²/ g.

### Beispiel 11

5,22 g VO(H₂PO₄)₂-Ausgangsprodukt und 1,91 g ZrO(OH)₂ wurden durch Mörsern intensiv gemischt und wie in Beispiel 1 zu Splitt verarbeitet. 3,08 g Splitt wurden mit geringen Mengen Wasser befeuchtet und in einer geschlossenen Ampulle bei 130°C erhitzt. Nach Trocknen bei 130°C wurden 2,91 g Precursor, bestehend aus VOHPO₄ · 0,5H₂O und Zr-Phosphat, erhalten.
BET-Oberfläche= 10,1 m²/ g.

Der Katalysator wurde durch 2-stündiges Erhitzen bei 500°C hergestellt. BET-Oberfläche= 8,5 m²/ g.

### Beispiel 12

Der nach Beispiel 2 hergestellte Precursor wurde durch 2-stündiges Calcinieren bei 480°C im N₂-Strom in den Katalysator (VO)₂P₂O₇ · BiPO₄ umgewandelt. Von diesem Katalysator wurden 6,0 g Splitt in einem Festbettreaktor zur Reaktion eines Butan/ Luft-Gemisches (1,5 Vol% Butan, GHSV 1000 h⁻¹) bei 450°C eingesetzt. Bei einem Butanumsatz von 50% wurde eine Maleinsäureanhydridausbeute von 37 Mol-% erreicht.

### Beispiel 13

Die Siebfraktion von 1-1,25 mm des nach Beispiel 2 oder 3 hergestellten Katalysators wurde für die Ammoxidation von Toluol zu Benzonitril eingesetzt. Folgende Bedingungen wurden angewendet: Toluol : Ammoniak : Luft : Wasserdampf =1 : 4,5 : 32 : 24, Normaldruck, T_{Reaktion} = 350°C, reziproke Katalysatorbelastung W/F = ca. 10 g h mol⁻¹. Der Toluolumsatz betrug 15 Mol-% und die Benzonitrilausbeute erreichte 13 Mol-%. Das entspricht einer Benzonitrilselektivität von ca. 87%.

### Beispiel 14

Eine Siebfraktion von 1-1,25 mm des nach Beispiel 2 hergestellten Katalysators wurde für die Oxidation von Toluol zu Benzaldehyd eingesetzt. Folgende Bedingungen wurden angewendet: Toluol : Luft = 1 : 100, Normaldruck, T_{Reaktion} = 410°C, reziproke Katalysatorbelastung W/F = ca. 1,3 g h mol⁻¹. Der Toluolumsatz lag bei 5 Mol-% und die Benzaldehydausbeute bei 1,5 Mol-%. Das entspricht einer Benzaldehydselektivität von ca. 30%

## Patentansprüche

1. Mischkatalysator aus Oxovanadiumdiphosphat und weiteren Komponenten, **dadurch gekennzeichnet, dass** der Katalysator die allgemeine Formel (I)
(VO)₂P₂O₇· A (I)
aufweist, worin A die Bedeutung Meₓ(PO₄)_{y} oder Me₂P₂O₇ hat und x = 1 bis 3 und y = 1, 2 und 4 darstellen, und Me ein Metall ist, ausgewählt aus der Gruppe, die aus Fe, La, Lanthaniden, Sb, Al, Bi, Zr, Zn, Mn, Cu, Sn und Ti besteht,
und der Katalysator hergestellt ist durch Reaktion von VO(H₂PO₄)₂ mit einem Metalloxid, Metallhydroxid, Metalloxidhydroxid oder einem Gemisch davon oder eines Gemisches von Me(H₂PO₄)₂ und VO₂ unter hydrothermalen Bedingungen in einem geschlossenen System in Anwesenheit von 0,5 bis 40 Mol-% Wasser bei einem Druck von 1,05 bis 6 bar und bei einer Temperatur im Bereich von 100 bis 150°C über einen Zeitraum von 0,5 bis 100 Stunden zu VOHPO₄ · 0,5H₂O und einem Metallphosphatprodukt, wobei das Molverhältnis von Metalloxid zu VO(H₂PO₄)₂ von 0,9:1 bis 1,1:2 beträgt, und das erhaltene Gemisch bei Temperaturen von 380 bis 700°C calciniert wird bis zur Bildung des Katalysators der Formel (I).

2. Katalysator nach Anspruch 1, **dadurch gekennzeichnet, dass** das Metall Titan, Lanthan, Kupfer, Aluminium, Zirkonium oder Eisen ist.

3. Katalysator nach Anspruch 1, **dadurch gekennzeichnet, dass** die Herstellung durch Reaktion von VO(H₂PO₄)₂ mit einem oder mehreren Metalloxid(en) oder Metallhydroxid(en) erfolgt.

4. Katalysator nach Anspruch 1, **dadurch gekennzeichnet, dass** das Metallphosphatprodukt kristallwasserhaltig ist.

5. Verwendung des Katalysators nach einem der Ansprüche 1 bis 4 zur Oxidation von Butan zu Maleinsäureanhydrid.

6. Verwendung des Katalysators nach einem der Ansprüche 1 bis 4 zur Oxidation bzw. Ammoxidation von Alkylaromaten zu den entsprechenden Aldehyden bzw. Nitrilen.
